# EUROPÄISCHE PATENTANMELDUNG

(11) **EP 4 042 958 A1**
(43) Veröffentlichungstag der Anmeldung: **17.08.2022**
(21) Anmeldenummer: 22156749.8
(22) Anmeldetag: 15.02.2022
(51) Int. Cl.: A61B 17/86, A61B 17/88

(54) **MEDIZINISCHE DREHMOMENTBEGRENZUNGSVORRICHTUNG**

(30) Priorität: 15.02.2021 DE 102021103460
(71) Anmelder: Aesculap AG, 78532 Tuttlingen (DE)
(72) Erfinder: Lindner, Stephan, 78573 Wurmlingen (DE)
(74) Vertreter: Hoeger, Stellrecht & Partner Patentanwälte mbB

(57) **Zusammenfassung**

Um eine medizinische Drehmomentbegrenzungsvorrichtung, welche eine Längsachse definiert und eine erste Komponente und eine zweite Komponente umfasst, wobei die erste Komponente und die zweite Komponente zusammenwirkend ausgebildet sind zum Übertragen eines auf die erste Komponente wirkenden Drehmoments auf die zweite Komponente derart, dass bei einem in einer Anzugsdrehrichtung auf die erste Komponente wirkenden Drehmoment, welches kleiner als ein Grenzdrehmoment ist, die erste Komponente und die zweite Komponente in der Anzugsdrehrichtung relativ zueinander um die Längsachse unverdrehbar und bei einem in der Anzugsdrehrichtung auf die erste Komponente wirkenden Drehmoment, welches mindestens dem Grenzdrehmoment entspricht, die erste Komponente und die zweite Komponente in der Anzugsdrehrichtung relativ zueinander um die Längsachse verdrehbar sind, so zu verbessern, dass mit hoher Präzision auf einfache Weise herstellbar ist, wird vorgeschlagen, dass an der ersten Komponente mindestens ein verformbares Mitnehmerelement angeordnet oder ausgebildet ist, dass an der zweiten Komponente eine mit dem Mitnehmerelement zusammenwirkende Verformungsfläche angeordnet oder ausgebildet ist und dass sich ein Abstand der Verformungsfläche von der Längsachse in Umfangsrichtung ändert.

## Beschreibung

Die vorliegende Erfindung betrifft eine medizinische Drehmomentbegrenzungsvorrichtung, welche eine Längsachse definiert und eine erste Komponente und eine zweite Komponente umfasst, wobei die erste Komponente und die zweite Komponente zusammenwirkend ausgebildet sind zum Übertragen eines auf die erste Komponente wirkenden Drehmoments auf die zweite Komponente derart, dass bei einem im einer Anzugsdrehrichtung auf die erste Komponente wirkenden Drehmoment, welche kleiner als ein Grenzdrehmoment ist, die erste Komponente und die zweite Komponente in der Anzugsdrehrichtung relativ zueinander um die Längsachse unverdrehbar und bei einem in der Anzugsdrehrichtung auf die erste Komponente wirkenden Drehmoment, welches mindestens den Grenzdrehmoment entspricht, die erste Komponente und die zweite Komponente in der Anzugsdrehrichtung relativ zueinander um die Längsachse verdrehbar sind.

Medizinische Drehmomentbegrenzungsvorrichtungen sind in unterschiedlichen Ausgestaltungen bekannt. Beispielsweise ist in der DE 10 2018 105 682 A1 eine Drehmomentbegrenzungseinrichtung in Form einer medizinischen Mutter offenbart.

Ein Problem bei bekannten Drehmomentbegrenzungsvorrichtungen ist insbesondere, dass das Grenzdrehmoment sehr stark von Fertigungstoleranzen von deren Komponenten abhängig ist. Insbesondere bei bei medizinischen Vorrichtungen wie Implantaten ist ein Überschreiten eines vordefinierten Grenzdrehmoments unbedingt zu vermeiden. Um eine Beschädigung beispielsweise eines Implantats während eines chirurgischen Eingriffs aufgrund einer Überschreitung des Grenzdrehmoments zu vermeiden, müssen die Komponenten der Drehmomentbegrenzungsvorrichtung mit sehr hoher Präzision hergestellt werden. Mithin ist es also bei bekannten medizinischen Drehmomentbegrenzungsvorrichtungen erforderlich, einen großen Aufwand zu betreiben, um Fertigungstoleranzen der zusammenwirkenden Komponenten möglichst gering zu halten.

Es ist daher eine Aufgabe der vorliegenden Erfindung, eine medizinische Drehmomentbegrenzungsvorrichtung mit hoher Präzision auf einfache Weise herzustellen.

Diese Aufgabe wird bei einer medizinischen Drehmomentbegrenzungsvorrichtung der eingangs beschriebenen Art erfindungsgemäß dadurch gelöst, dass an der ersten Komponente mindestens ein verformbares Mitnehmerelement angeordnet oder ausgebildet ist, dass an der zweiten Komponente eine mit dem Mitnehmerelement zusammenwirkende Verformungsfläche angeordnet oder ausgebildet ist und dass sich ein Abstand der Verformungsfläche von der Längsachse in Umfangsrichtung ändert.

Die vorgeschlagene Weiterbildung ermöglicht es insbesondere, ein Grenzdrehmoment für die medizinischen Drehmomentbegrenzungsvorrichtung mit sehr hoher Präzision vorzugeben. Dies wird insbesondere erreicht durch Auswahl eines zur ersten Komponente und zur zweiten Komponente passenden Mitnehmerelements, welches verformbar ausgebildet ist. Die Drehmomentbegrenzungsvorrichtung kann insbesondere auch mehrere solcher verformbaren Mitnehmerelemente umfassen. Durch separate Ausbildung der ersten Komponente und des mindestens einen Mitnehmerelements kann dann das passende Mitnehmerelement aus einer Mehrzahl von ausgebildeten Mitnehmerelementen ausgewählt und an der ersten Komponente angeordnet werden. Werden insbesondere mehrere Mitnehmerelemente vorgesehen, können diese unter Berücksichtigung der Fertigungstoleranzen sowohl der ersten Komponente, der zweiten Komponente und aller verfügbaren Mitnehmerelemente derart ausgewählt werden, dass das gewünschte Grenzdrehmoment mit hoher Präzision vorgebbar ist. Zum Beispiel kann das Grenzdrehmoment durch drei Mitnehmerelemente vorgegeben. Ist beispielsweise das durch das erste Mitnehmerelement vorgegebene Grenzdrehmoment aufgrund von Fertigungstoleranzen etwas zu gering, kann zum Ausgleich ein zweites Mitnehmerelement gewählt werden, dessen Grenzdrehmoment aufgrund von Fertigungstoleranzen etwas zu groß ist. Wird hierzu noch ein passendes drittes Mitnehmerelement gewählt, dessen Abweichung vom gewünschten, also vorzugebenden Grenzdrehmoment für die Drehmomentbegrenzungsvorrichtung insgesamt der Summe der Abweichungen der beiden anderen Mitnehmerelemente entspricht, kann so allein durch Auswahl unterschiedlicher Mitnehmerelemente, die jeweils mit Fertigungstoleranzen behaftet sind, insgesamt jedoch eine medizinische Drehmomentbegrenzungsvorrichtung ausgebildet werden, die das Grenzdrehmoment hochpräzise vorgibt. Die vorgeschlagene separate Ausbildung des verformbaren Mitnehmerelements, welches im Zusammenwirken mit der Verformungsfläche an der zweiten Komponente beim Einleiten eines Drehmoments in die erste Komponente bis zum Erreichen des Grenzdrehmoments verformt wird, ermöglicht so allein durch Auswahl eines aufgrund seiner konkreten Fertigungstoleranz optimal zur Einstellung des Grenzdrehmoments geeigneten Mitnehmerelements, dass Fertigungstoleranzen im Zusammenwirken aller Komponenten, wenn sie nicht vollständig ausgeglichen werden können, zumindest minimiert werden können.

Günstig ist es, wenn dass das mindestens eine Mitnehmerelement mindestens einen Verformungsabschnitt umfasst, wenn am mindestens einen Verformungsabschnitt eine mit der Verformungsfläche zusammenwirkende Anlagefläche angeordnet oder ausgebildet ist und wenn der mindestens einen Verformungsabschnitt Verformungsabschnitt ausgehend von einer Grundstellung, in welcher kein Drehmoment auf die erste Komponente wirkt, in eine Auslösestellung verformbar ist, in welcher auf die erste Komponente das Grenzdrehmoment wirkt. Diese Ausgestaltung ermöglicht es insbesondere, dass mindestens eine Mitnehmerelement in gewünschter Weise auszubilden. Dabei wird nicht das gesamte Mitnehmerelement im Zusammenwirken mit der zweiten Komponente verformt, sondern lediglich mindestens ein Verformungsabschnitt desselben. Das mindestens eine Mitnehmerelement kann zum Beispiel einen Verformungsabschnitt oder auch mehrere Verformungsabschnitte umfassen.

Die vorgeschlagene Drehmomentbegrenzungsvorrichtung ermöglicht insbesondere unterschiedlichste Ausgestaltungen. Beispielsweise kann die zweite Komponente derart ausgebildet sein, dass die Verformungsfläche in Richtung auf die Längsachse hin oder alternativ in einer Richtung von der Längsachse weg weist. Entsprechend ist dann das Mitnehmerelement ausgebildet, beispielsweise mit einem von der Längsachse weg weisenden Verformungsabschnitt oder mit einem in Richtung auf die Längsachse hin weisenden mindestens einen Verformungsabschnitt.

Günstigerweise ist der mindestens eine Verformungsabschnitt in der Auslösestellung maximal verformt. So kann auf einfache Weise ein Grenzdrehmoment für das jeweilige Mitnehmerelement vorgegeben werden.

Um die medizinische Drehmomentbegrenzungsvorrichtung mehrfach nutzen zu können, ist es vorteilhaft, wenn der mindestens eine Verformungsabschnitt ein federelastisches Element umfasst oder in Form eines federelastischen Elements ausgebildet ist.

Günstigerweise ist der Verformungsabschnitt in radialer Richtung bezogen auf die Längsachse federnd ausgebildet. Dies ermöglicht es insbesondere, den Verformungsabschnitt zu verformen im Zusammenwirken mit der Verformungsfläche, wenn sich deren Abstand von der Längsachse in Umfangsrichtung ändert.

Gemäß einer weiteren bevorzugten Ausführungsform der Erfindung kann vorgesehen sein, dass der mindestens eine Verformungsabschnitt einen Verformungsvorsprung umfasst, dass der Verformungsvorsprung die Anlagefläche umfasst und bezogen auf die Längsachse in Richtung auf die Verformungsfläche vorstehend oder vorgewölbt ausgebildet ist. Diese Ausgestaltung ermöglicht es insbesondere, dass lediglich der mindestens eine Verformungsabschnitt mit der Verformungsfläche in Kontakt treten kann. Fertigungstoleranzen bei der Herstellung der ersten Komponente spielen für die Drehmomentbegrenzungsvorrichtung in diesem Fall praktisch keine Rolle. Fertigungstoleranzen der Drehmomentbegrenzungsvorrichtung werden auf diese Weise ausschließlich bestimmt durch Fertigungstoleranzen im Bereich des mindestens einen Verformungsabschnitts und der mit diesem zusammenwirkenden Verformungsfläche an der zweiten Komponente.

Vorteilhaft ist es, wenn die die Verformungsfläche mindestens einen ersten Flächenbereich und mindestens einen zweiten Flächenbereich umfasst, wenn der erste Flächenbereich mit dem mindestens einen Verformungsabschnitt in der Anzugsrichtung zusammenwirkend ausgebildet ist und wenn der mindestens eine zweite Flächenbereich in einer der Anzugsrichtung entgegengesetzt gerichteten Lösedrehrichtung mit dem mindestens einen Verformungsabschnitt zusammenwirkend ausgebildet ist. Diese Ausgestaltung ermöglicht es insbesondere, ein Grenzdrehmoment in Anzugsrichtung durch den mindestens einen ersten Flächenbereich im Zusammenwirken mit dem mindestens einen Verformungsabschnitt vorzugeben. Eigenschaften beim Lösen, nämlich beim Einleiten eines Lösemoments, welches in die Lösedrehrichtung wirkt, in die erste Komponente, können dann durch das Zusammenwirken des mindestens einen Verformungsabschnitts mit dem mindestens einen zweiten Flächenbereich vorgegeben werden.

Günstig ist es, wenn der mindestens eine erste Flächenbereich eine in Umfangsrichtung wirkende erste Anlauframpe bezogen auf die Längsachse für den mindestens einen Verformungsabschnitt bildet, wenn der mindestens eine zweite Flächenbereich eine in Umfangsrichtung wirkende zweite Anlauframpe bezogen auf die Längsachse für den mindestens einen Verformungsabschnitt bildet und wenn die erste Anlauframpe flacher ist als die zweite Anlauframpe. Die also im Vergleich zur ersten Anlauframpe steilere zweite Anlauframpe ermöglicht es insbesondere, ein Lösemoment in die erste Komponente einzuleiten und so beispielsweise eine Schraubverbindung oder dergleichen wieder zu lösen, und zwar sicher und mit einer hohen Wahrscheinlichkeit, dass das Grenzdrehmoment beim Lösen nicht überschritten wird.

Vorteilhaft ist es, wenn die Verformungsfläche mehrere erste und zweite Flächenbereiche umfasst und wenn die ersten und zweiten Flächenbereiche in Umfangsrichtung abwechselnd angeordnet oder ausgebildet sind. Eine solche Ausgestaltung erleichtert insbesondere eine Montage der medizinischen Drehmomentbegrenzungsvorrichtung. Zudem ist es beispielsweise möglich, die medizinische Drehmomentbegrenzungsvorrichtung mehrfach nacheinander durch Einleiten eines Drehmoments in Anzugsrichtung auszulösen, welches das Grenzdrehmoment überschreitet. Der Verformungsabschnitt kann sich nach Überschreiten des Grenzdrehmoments beispielsweise wieder in eine Ausgangsstellung zurückverformen, wenn er in den Bereich der zweiten Anlauframpe gelangt.

Günstigerweise entspricht eine Anzahl M der ersten Flächenbereiche mindestens einer Anzahl N der Verformungsabschnitte. Insbesondere ist die Anzahl M ein ganzzahliges Vielfaches der Anzahl N. Dies erleichtert eine Montage der Drehmomentbegrenzungsvorrichtung und ermöglicht insbesondere auch ein mehrfaches Auslösen derselben, wenn die erste Komponente um eine volle Umdrehung um die Längsachse verdreht wird.

Einfach und kompakt lässt sich die medizinische Drehmomentbegrenzungsvorrichtung ausbilden, wenn die Verformungsfläche in Richtung auf die Längsachse hin weisend ausgebildet ist. Wie bereits oben erwähnt ist es alternativ auch möglich, die zweite Komponente derart auszubilden, dass die Verformungsfläche in einer Richtung von der Längsachse weg weisend ausgebildet ist.

Vorteilhafterweise ist der mindestens eine Verformungsabschnitt durch eine Materialschwächung am mindestens einen Mitnehmerelement ausgebildet. So kann eine Verformbarkeit des mindestens einen Mitnehmerelements auf einfache Weise vorgegeben werden.

Gemäß einer weiteren bevorzugten Ausführungsform der Erfindung kann vorgesehen sein, dass die Drehmomentbegrenzungsvorrichtung mehrere Mitnehmerelemente umfasst. Insbesondere kann sie 2 bis 20 Mitnehmerelemente umfassen. Weiter insbesondere kann die Drehmomentbegrenzungsvorrichtung 6 bis 14 Mitnehmerelemente umfassen. Wie eingangs eingehend erläutert, kann ein Grenzdrehmoment der Drehmomentbegrenzungsvorrichtung insbesondere dadurch vorgegeben werden, dass die Grenzdrehmomente, die die einzelnen Mitnehmerelemente vorgeben, addiert werden. Durch entsprechende Wahl der mehreren Mitnehmerelemente ist es so auf einfache Weise möglich, Fertigungstoleranzen bei der Herstellung der einzelnen Mitnehmerelemente zu kompensieren. So lässt sich das Grenzdrehmoment der Drehmomentbegrenzungsvorrichtung insgesamt auf einfache und hochpräzise Weise vorgeben.

Die Ausbildung und Montage der Drehmomentbegrenzungsvorrichtung kann insbesondere dadurch weiter vereinfacht werden, dass die mehreren Mitnehmerelemente identisch ausgebildet sind.

Die Herstellung der Drehmomentbegrenzungsvorrichtung kann insbesondere dadurch weiter vereinfacht werden, dass das mindestens eine Mitnehmerelement spiegelsymmetrisch bezogen auf eine Längsachse enthaltende erste Spiegelebene ausgebildet ist.

Vorzugsweise ist das mindestens eine Mitnehmerelement mit der ersten Komponente lösbar verbindbar ausgebildet. Dies ermöglicht es insbesondere, ein Mitnehmerelement gegebenenfalls auszutauschen, beispielsweise dann, wenn es sich bei der Prüfung der Drehmomentbegrenzungsvorrichtung ergeben sollte, dass das Grenzdrehmoment nicht dem vorgegebenen Wert entspricht.

Auf einfache Weise kann das mindestens eine Mitnehmerelement mit der ersten Komponente lösbar verbunden werden, wenn die medizinische Drehmomentbegrenzungsvorrichtung eine Kopplungseinrichtung umfasst zum drehfesten Koppeln des mindestens einen Mitnehmerelements und der ersten Komponente in einer Kopplungsstellung.

Die Drehmomentbegrenzungsvorrichtung kann insbesondere auf einfache Weise ausgebildet werden, wenn die Kopplungseinrichtung erste und zweite Kopplungselemente umfasst, wenn mindestens ein Kopplungselement der ersten und zweiten Kopplungselemente am Mitnehmerelement angeordnet oder ausgebildet ist, wenn mindestens ein Kopplungselement der ersten und zweiten Kopplungselemente an der ersten Komponente angeordnet oder ausgebildet ist und wenn die ersten und zweiten Kopplungselemente, die am Mitnehmerelement einerseits und an der ersten Komponente andererseits angeordnet oder ausgebildet sind, in der Kopplungsstellung kraft- und/oder formschlüssig in Eingriff stehen. Zum Koppeln des mindestens einen Mitnehmerelements und der ersten Komponente müssen also lediglich die ersten und zweiten Kopplungselemente derselben miteinander kraft- und/oder formschlüssig in Eingriff gebracht werden. Eine Kopplung ist besonders einfach, wenn sie ausschließlich formschlüssig erfolgt.

Auf einfache Weise lässt sich die Kopplungseinrichtung ausbilden, wenn das erste Kopplungselement in Form eines Kopplungsvorsprungs ausgebildet ist und wenn das zweite Kopplungselement in Form einer zum Kopplungsvorsprung korrespondierenden Kopplungsaufnahme ausgebildet ist.

Um insbesondere eine optimale drehfeste Kopplung des Mitnehmerelements und der ersten Komponente zu erreichen, ist es günstig, wenn der Kopplungsvorsprung in Richtung auf die Längsachse hin weisend vorsteht und wenn die korrespondierende Kopplungsaufnahme von der Längsachse weg weisend geöffnet ist. Alternativ kann der Kopplungsvorsprung auch in Richtung von der Längsachse weg weisend vorstehen und die korrespondierende Kopplungsaufnahme in Richtung auf die Längsachse hin weisend geöffnet sein.

Um insbesondere mehrere Mitnehmerelemente mit der ersten Komponente schnell und einfach koppeln zu können, ist es vorteilhaft, wenn die Kopplungsaufnahme in Form einer sich parallel zur Längsachse erstreckenden Kopplungsnut ausgebildet ist. So können beispielsweise mehrere Mitnehmerelemente, die aus einem Flachmaterial ausgebildet sind, in Form eines Mitnehmerelementpakets direkt aneinander anliegend mit der ersten Komponente gekoppelt werden.

Vorteilhaft ist es, wenn die Kopplungsnut ein erstes und ein zweites Ende aufweist, wenn das erste Ende geschlossen ist und wenn das zweite Ende offen ist zum Einführen eines Kopplungsvorsprungs parallel zur Längsachse. Diese Ausgestaltung vereinfacht eine Montage der medizinischen Drehmomentbegrenzungsvorrichtung. Durch das geschlossene erste Ende wird eine Bewegung des mindestens einen Mitnehmerelements relativ zur ersten Komponente in einer Richtung parallel zur Längsachse auf einfache Weise begrenzt.

Die erste Komponente kann unterschiedliche Abschnitte umfassen, beispielsweise einen Abschnitt, in dem ein Drehmoment eingeleitet wird. Ferner ist es günstig, wenn die erste Komponente einen Kopplungsabschnitt umfasst, welcher sich parallel zur Längsachse erstreckt, und wenn die von der ersten Komponente umfassten Kopplungselemente im Bereich des Kopplungsabschnitts angeordnet oder ausgebildet sind, insbesondere aussschließlich. Diese Ausgestaltung ermöglicht insbesondere eine definierte Positionierung des mindestens einen Mitnehmerelements an der ersten Komponente, und zwar im Bereich des Kopplungsabschnitts.

Auf einfache Weise lässt sich die medizinische Drehmomentbegrenzungsvorrichtung ausbilden, wenn der Kopplungsabschnitt spiegelsymmetrisch bezogen auf eine die Längsachse enthaltende zweite Spiegelebene ausgebildet ist. Dies vereinfacht insbesondere eine Montage der medizinischen Drehmomentbegrenzungsvorrichtung, insbesondere das Anordnen des mindestens einen Mitnehmerelements an der ersten Komponente.

Günstig ist es, wenn der Kopplungsabschnitt N Kopplungselemente umfasst und wenn eine Symmetrie des Kopplungsabschnitts bezogen auf die Längsachse entsprechend einer Anzahl N der Kopplungselemente N-zählig ist. Dies ermöglicht es insbesondere, das mindestens eine Mitnehmerelement in unterschiedlichen Drehstellungen bezogen auf die Längsachse mit der ersten Komponente in Eingriff zu bringen. Mithin wird also eine Montage der medizinischen Drehmomentbegrenzungsvorrichtung auf diese Weise vereinfacht.

Günstigerweise sind am mindestens einen Mitnehmerelement in Umfangsrichtung abwechselnd ein Kopplungselement und ein Verformungsabschnitt angeordnet oder ausgebildet sind. So können insbesondere unterschiedliche Funktionsbereiche am Mitnehmerelement ausgebildet werden, nämlich Kopplungselemente zum Koppeln mit der ersten Komponente und Verformungsabschnitte zum Zusammenwirken mit der zweiten Komponente. Eine solche Trennung ermöglicht insbesondere eine Verbesserung einer Präzision der medizinischen Drehmomentbegrenzungsvorrichtung.

Vorteilhafterweise ist das mindestens eine Mitnehmerelement in Form eines in sich geschlossenen, die Längsachse umgebenden Ringelements ausgebildet. Insbesondere kann das mindestens eine Mitnehmerelement aus einem Flachmaterial ausgebildet sein. Dies ermöglicht es insbesondere, mehrere Mitnehmerelemente direkt aneinander in Anlage zu bringen und beispielsweise in Form eines Mitnehmerelementpakets an der ersten Komponente anzuordnen, beispielsweise am Kopplungsabschnitt der ersten Komponente.

Gemäß einer weiteren bevorzugten Ausführungsform der Erfindung kann vorgesehen sein, dass die erste Komponente und die zweite Komponente in einer Sicherungsstellung unverlierbar aneinander gesichert sind. So können insbesondere keine Teile der Drehmomentbegrenzungsvorrichtung verloren gehen. Dies sollte insbesondere bei einem chirurgischen Eingriff im Körper eines Patienten unbedingt vermieden werden. Insbesondere können die aneinander in der Sicherungsstellung gesicherten ersten und zweiten Komponenten automatisch auch das mindestens eine Mitnehmerelement an der ersten Komponente gegen ein unbeabsichtigtes Lösen sichern.

Auf einfache Weise lässt sich die medizinische Drehmomentbegrenzungsvorrichtung ausbilden, wenn sie eine Sicherungseinrichtung zum Sichern der ersten Komponente und der zweiten Komponente in der Sicherungsstellung gegen eine Bewegung relativ zueinander in einer Richtung parallel zur Längsachse umfasst. Insbesondere dann, wenn die erste Komponente und die zweite Komponente einander umgebend angeordnet oder ausgebildet sind, kann so ein unerwünschtes Trennen derselben verhindert werden.

Günstigerweise ist die Sicherungseinrichtung ausgebildet zum Begrenzen einer Bewegung des mindestens einen Mitnehmerelements und der ersten Komponente in einer Richtung parallel zur Längsachse. So kann mit der Sicherungseinrichtung insbesondere sichergestellt werden, dass das mindestens eine Mitnehmerelement an der ersten Komponente in definierter Weise positioniert verbleibt.

Vorteilhaft ist es, wenn die Sicherungseinrichtung zwei aufeinander zu weisende Anschläge für das mindestens eine Mitnehmerelement umfasst, wobei insbesondere ein erster Anschlag an der ersten Komponente angeordnet oder ausgebildet ist und ein zweiter Anschlag an der zweiten Komponente angeordnet oder ausgebildet ist. So kann das mindestens eine Mitnehmerelement an der Drehmomentbegrenzungsvorrichtung in definierter Weise positioniert werden, und zwar zwischen den zwei Anschlägen. Je nachdem, wie groß der Abstand der beiden Anschläge voneinander ist und wie viele Mitnehmerelemente vorgesehen sind, kann so das mindestens eine Mitnehmerelement sich zwischen den zwei Anschlägen in einer Richtung parallel zur Längsachse bewegen oder auch nicht.

Auf einfache Weise kann die Drehmomentbegrenzungsvorrichtung ausgebildet werden, wenn einer der Anschläge in Form eines in radialer Richtung vorspringenden Ringflansches ausgebildet ist. Dieser kann insbesondere an der ersten Komponente angeordnet oder ausgebildet sein. Beispielsweise kann er sich an das geschlossene Ende der Kopplungsnut anschließen.

Einfach und kompakt lässt sich die Drehmomentbegrenzungsvorrichtung realisieren, wenn einer der Anschläge durch das geschlossene Ende der Kopplungsnut gebildet ist.

Günstig ist es, wenn die Sicherungseinrichtung mindestens ein Sicherungselement umfasst zum Begrenzen einer Bewegung der ersten Komponente und der zweiten Komponente in einer Richtung parallel zur Längsachse. Das mindestens eine Sicherungselement kann insbesondere auch derart ausgebildet sein, dass eine Bewegung der ersten Komponente und der zweiten Komponente relativ zueinander parallel zur Längsachse vollständig unterbunden wird.

Auf einfache Weise lässt sich die Sicherungseinrichtung ausbilden, wenn das mindestens eine Sicherungselement einen Sicherungsring umfasst und wenn der Sicherungsring in der Sicherungsstellung in die Längsachse umgebende Ringaufnahmen an der ersten und der zweiten Komponente eingreift. Die beiden Ringaufnahmen sind einerseits in einer Richtung auf die Längsachse hin weisend und andererseits in einer Richtung von der Längsachse weg weisend geöffnet, sodass der Sicherungsring gleichzeitig in beide Ringaufnahmen eingreifen kann. So kann eine Bewegung der ersten Komponente und der zweiten Komponente relativ zueinander parallel zur Längsachse verhindert werden.

Um eine einfache Montage der medizinischen Drehmomentbegrenzungsvorrichtung zu ermöglichen, ist es günstig, wenn der Sicherungsring offen ausgebildet ist und zwei aufeinander zu weisende Sicherungsringenden aufweist. Beispielsweise kann er so in Form eines Sprengrings ausgebildet sein, dessen Außendurchmesser bei der Montage temporär verändert werden kann, und welcher dann wieder in seiner ursprünglichen Form in beide Ringaufnahmen gleichzeitig eingreifen kann.

Damit der Sicherungsring insbesondere auch eine Bewegung des mindestens einen Mitnehmerelements begrenzen kann, ist es günstig, wenn die Ringaufnahme an der ersten Komponente und die Kopplungsnut sich kreuzen.

Um auf einfache Weise ein Drehmoment in die erste Komponente einleiten zu können, ist es vorteilhaft, wenn an der ersten Komponente ein Werkzeugkopplungsglied angeordnet oder ausgebildet ist zum kraft- und/oder formschlüssigen in Eingriff Bringen mit einem ein Drehmoment in die erste Komponente einleitenden medizinischen Werkzeug. So kann ein Drehmoment in definierter Weise eingeleitet werden.

Auf einfache Weise lässt sich die Drehmomentbegrenzungsvorrichtung ausbilden und handhaben, wenn das Werkzeugkopplungsglied in Form eines Werkzeugkopplungsvorsprungs oder in Form einer Werkzeugkopplungsaufnahme ausgebildet ist.

Eine einfache Handhabung der Drehmomentbegrenzungsvorrichtung ist insbesondere möglich, wenn die Werkzeugkopplungsaufnahme schlitz-, kreuz- oder sternförmig ausgebildet ist.

Die Drehmomentbegrenzungsvorrichtung lässt sich insbesondere auf einfache handhaben, wenn eine Kontur des Werkzeugkopplungsglieds in Form eines Vielkant oder in Form eines Vielrund ausgebildet ist. Insbesondere können sowohl Werkzeugkopplungsvorsprünge als auch Werkzeugkopplungsaufnahmen eine derartige Kontur aufweisen.

Um insbesondere eine Drehmomentbegrenzungsmutter ausbilden zu können, ist es günstig, wenn an der zweiten Komponente ein Gewindeabschnitt angeordnet oder ausgebildet ist. Insbesondere kann der Gewindeabschnitt in Form eines Innengewindes oder eines Außengewindes ausgebildet sein. Eine Drehmomentbegrenzungsmutter kann insbesondere ausgebildet werden mit einem Innengewinde. Eine medizinische Schraube mit Drehmomentbegrenzungseinrichtung kann insbesondere mit einem Außengewindepunkt ausgebildet werden.

Für einen kompakten Aufbau der medizinischen Drehmomentbegrenzungsvorrichtung ist es vorteilhaft, wenn die erste Komponente und/oder die zweite Komponente hülsenförmig ausgebildet sind. So können sie insbesondere einander abschnittsweise umgebend ausgebildet werden.

Für den Einsatz bei medizinischen, insbesondere chirurgischen, Eingriffen ist es günstig, wenn die Drehmomentbegrenzungsvorrichtung aus einem oder mehreren sterilisierbaren Werkstoffen ausgebildet ist. So kann insbesondere sichergestellt werden, dass die medizinische Drehmomentbegrenzungsvorrichtung keimfrei und steril eingesetzt werden kann.

Gemäß einer weiteren bevorzugten Ausführungsform der Erfindung kann vorgesehen sein, dass die medizinische Drehmomentbegrenzungsvorrichtung in Form einer medizinischen Mutter, einer medizinischen Schraube oder eines medizinischen Drehmomentbegrenzungsschlüssels ausgebildet ist. Je nachdem, welcher Typ der Drehmomentbegrenzungsvorrichtung realisiert werden soll, werden die erste Komponente und die zweite Komponente entsprechend ausgebildet, beispielsweise bei einer Mutter mit einem Innengewinde an der zweiten Komponente und einem Werkzeugkopplungsvorsprung, insbesondere in Form eines Außenvielkant, an der ersten Komponente. Eine medizinische Schraube kann beispielsweise mit einer Werkzeugkopplungsaufnahme in Form eines Schlitzes an der ersten Komponente und mit einem Außengewinde an der zweiten Komponente ausgebildet werden.

Die vorstehende Beschreibung umfasst somit insbesondere die nachfolgend in Form durchnummerierter Sätze definierten Ausführungsformen medizinischer Drehmomentbegrenzungsvorrichtungen:
1. Medizinische Drehmomentbegrenzungsvorrichtung (10), welche eine Längsachse (12) definiert und eine erste Komponente (14) und eine zweite Komponente (16) umfasst, wobei die erste Komponente (14) und die zweite Komponente (16) zusammenwirkend ausgebildet sind zum Übertragen eines auf die erste Komponente (14) wirkenden Drehmoments auf die zweite Komponente (16) derart, dass bei einem in einer Anzugsdrehrichtung (18) auf die erste Komponente (14) wirkenden Drehmoment, welches kleiner als ein Grenzdrehmoment ist, die erste Komponente (14) und die zweite Komponente (16) in der Anzugsdrehrichtung (18) relativ zueinander um die Längsachse (12) unverdrehbar und bei einem in der Anzugsdrehrichtung (18) auf die erste Komponente (14) wirkenden Drehmoment, welches mindestens dem Grenzdrehmoment entspricht, die erste Komponente (14) und die zweite Komponente (16) in der Anzugsdrehrichtung relativ zueinander um die Längsachse (12) verdrehbar sind, dadurch gekennzeichnet, dass an der ersten Komponente (14) mindestens ein verformbares Mitnehmerelement (20) angeordnet oder ausgebildet ist, dass an der zweiten Komponente (16) eine mit dem Mitnehmerelement (20) zusammenwirkende Verformungsfläche (22) angeordnet oder ausgebildet ist und dass sich ein Abstand (24) der Verformungsfläche (22) von der Längsachse (12) in Umfangsrichtung ändert.
2. Medizinische Drehmomentbegrenzungsvorrichtung nach Satz 1, dadurch gekennzeichnet, dass das mindestens eine Mitnehmerelement (20) mindestens einen Verformungsabschnitt (88) umfasst, dass am Verformungsabschnitt (88) eine mit der Verformungsfläche (22) zusammenwirkende Anlagefläche (90) angeordnet oder ausgebildet ist und dass der Verformungsabschnitt (88) ausgehend von einer Grundstellung, in welcher kein Drehmoment auf die erste Komponente (14) wirkt, in eine Auslösestellung verformbar ist, in welcher auf die erste Komponente (14) das Grenzdrehmoment wirkt.
3. Medizinische Drehmomentbegrenzungsvorrichtung nach Satz 2, dadurch gekennzeichnet, dass der mindestens eine Verformungsabschnitt (88) in der Auslösestellung maximal verformt ist.
4. Medizinische Drehmomentbegrenzungsvorrichtung nach Satz 2 oder 3, dadurch gekennzeichnet, dass der mindestens eine Verformungsabschnitt (88) ein federelastisches Element (92) umfasst oder in Form eines federelastischen Elements (92) ausgebildet ist.
5. Medizinische Drehmomentbegrenzungsvorrichtung nach einem der Sätze 2 bis 4, dadurch gekennzeichnet, dass der Verformungsabschnitt (88) in radialer Richtung bezogen auf die Längsachse (12) federnd ausgebildet ist.
6. Medizinische Drehmomentbegrenzungsvorrichtung nach einem der Sätze 2 bis 5, dadurch gekennzeichnet, dass der mindestens eine Verformungsabschnitt (88) einen Verformungsvorsprung (94) umfasst, dass der Verformungsvorsprung (94) die Anlagefläche (90) umfasst und bezogen auf die Längsachse (12) in Richtung auf die Verformungsfläche (22) vorstehend oder vorgewölbt ausgebildet ist.
7. Medizinische Drehmomentbegrenzungsvorrichtung nach einem der voranstehenden Sätze, dadurch gekennzeichnet, dass die Verformungsfläche (22) mindestens einen ersten Flächenbereich (66) und mindestens einen zweiten Flächenbereich (68) umfasst, dass der erste Flächenbereich (66) mit dem mindestens einen Verformungsabschnitt (88) in der Anzugsdrehrichtung (18) zusammenwirkend ausgebildet ist und dass der mindestens eine zweite Flächenbereich (68) in einer der Anzugsdrehrichtung (18) entgegengesetzt gerichteten Lösedrehrichtung (124) mit dem mindestens einen Verformungsabschnitt (88) zusammenwirkend ausgebildet ist.
8. Medizinische Drehmomentbegrenzungsvorrichtung nach Satz 7, dadurch gekennzeichnet, dass der mindestens eine erste Flächenbereich (66) eine in Umfangsrichtung wirkende erste Anlauframpe (78) bezogen auf die Längsachse (12) für den mindestens einen Verformungsabschnitt (88) bildet, dass der mindestens eine zweite Flächenbereich (68) eine in Umfangsrichtung wirkende zweite Anlauframpe (80) bezogen auf die Längsachse (12) für den mindestens einen Verformungsabschnitt (88) bildet und dass die erste Anlauframpe (78) flacher ist als die zweite Anlauframpe (80).
9. Medizinische Drehmomentbegrenzungsvorrichtung nach Satz 7 oder 8, dadurch gekennzeichnet, dass die Verformungsfläche (22) mehrere erste und zweite Flächenbereiche (66, 68) umfasst und dass die ersten und zweiten Flächenbereiche (66, 68) in Umfangsrichtung abwechselnd angeordnet oder ausgebildet sind.
10. Medizinische Drehmomentbegrenzungsvorrichtung nach Satz 9, dadurch gekennzeichnet, dass eine Anzahl M der ersten Flächenbereiche (66) mindestens einer Anzahl N der Verformungsabschnitte (88) entspricht, insbesondere einem ganzzahligen Vielfachen.
11. Medizinische Drehmomentbegrenzungsvorrichtung nach einem der voranstehenden Sätze, dadurch gekennzeichnet, dass die Verformungsfläche (22) in Richtung auf die Längsachse (12) hin weisend ausgebildet ist.
12. Medizinische Drehmomentbegrenzungsvorrichtung nach einem der voranstehenden Sätze, dadurch gekennzeichnet, dass der mindestens eine Verformungsabschnitt (88) durch eine Materialschwächung am mindestens einen Mitnehmerelement (20) ausgebildet ist.
13. Medizinische Drehmomentbegrenzungsvorrichtung nach einem der voranstehenden Sätze, dadurch gekennzeichnet, dass die Drehmomentbegrenzungsvorrichtung (10) mehrere Mitnehmerelemente (20) umfasst, insbesondere 2 bis 20 Mitnehmerelemente (20), weiter insbesondere 6 bis 14 Mitnehmerelemente (20).
14. Medizinische Drehmomentbegrenzungsvorrichtung nach Satz 13, dadurch gekennzeichnet, dass die mehreren Mitnehmerelemente (20) identisch ausgebildet sind.
15. Medizinische Drehmomentbegrenzungsvorrichtung nach einem der voranstehenden Sätze, dadurch gekennzeichnet, dass das mindestens eine Mitnehmerelement (20) spiegelsymmetrisch bezogen auf eine die Längsachse (12) enthaltende erste Spiegelebene (112) ausgebildet ist.
16. Medizinische Drehmomentbegrenzungsvorrichtung nach einem der voranstehenden Sätze, dadurch gekennzeichnet, dass das mindestens eine Mitnehmerelement (20) mit der ersten Komponente (14) lösbar verbindbar ausgebildet ist.
17. Medizinische Drehmomentbegrenzungsvorrichtung nach einem der voranstehenden Sätze, dadurch gekennzeichnet, dass die medizinische Drehmomentbegrenzungsvorrichtung (10) eine Kopplungseinrichtung (98) umfasst zum drehfesten Koppeln des mindestens einen Mitnehmerelements (20) und der ersten Komponente (14) in einer Kopplungsstellung.
18. Medizinische Drehmomentbegrenzungsvorrichtung nach Satz 17, dadurch gekennzeichnet, dass die Kopplungseinrichtung (98) erste und zweite Kopplungselemente (100, 102) umfasst, dass mindestens ein Kopplungselement (100) der ersten und zweiten Kopplungselemente (100, 102) am mindestens einen Mitnehmerelement (20) angeordnet oder ausgebildet ist, dass mindestens ein Kopplungselement (102) der ersten und zweiten Kopplungselemente (100, 102) an der ersten Komponente (14) angeordnet oder ausgebildet ist und dass die ersten und zweiten Kopplungselemente (110, 102), die am mindestens einen Mitnehmerelement (20) einerseits und an der ersten Komponente (14) andererseits angeordnet oder ausgebildet sind, in der Kopplungsstellung kraft- und/oder formschlüssig in Eingriff stehen.
19. Medizinische Drehmomentbegrenzungsvorrichtung nach Satz 18, dadurch gekennzeichnet, dass das erste Kopplungselement (100) in Form eines Kopplungsvorsprungs (104) ausgebildet ist und dass das zweite Kopplungselement (102) in Form einer zum Kopplungsvorsprungs (104) korrespondierenden Kopplungsaufnahme (40) ausgebildet ist.
20. Medizinische Drehmomentbegrenzungsvorrichtung nach Satz 19, dadurch gekennzeichnet, dass der Kopplungsvorsprung (104) in Richtung auf die Längsachse (12) hin weisend vorsteht und dass die korrespondierende Kopplungsaufnahme (40) von der Längsachse (12) weg weisend geöffnet ist.
21. Medizinische Drehmomentbegrenzungsvorrichtung nach Satz 19 oder 20, dadurch gekennzeichnet, dass die Kopplungsaufnahme (40) in Form einer sich parallel zur Längsachse (12) erstreckenden Kopplungsnut (38) ausgebildet ist.
22. Medizinische Drehmomentbegrenzungsvorrichtung nach Satz 21, dadurch gekennzeichnet, dass die Kopplungsnut (38) ein erstes und ein zweites Ende (106, 108) aufweist, dass das erste Ende (106) geschlossen ist und dass das zweite Ende (108) offen ist zum Einführen eines Kopplungsvorsprungs (104) parallel zur Längsachse (12).
23. Medizinische Drehmomentbegrenzungsvorrichtung nach einem der Sätze 18 bis 22, dadurch gekennzeichnet, dass die erste Komponente (14) einen Kopplungsabschnitt (26) umfasst, welcher sich parallel zur Längsachse (12) erstreckt, und dass die von der ersten Komponente (14) umfassten Kopplungselemente (102) im Bereich des Kopplungsabschnitts (26) angeordnet oder ausgebildet sind.
24. Medizinische Drehmomentbegrenzungsvorrichtung nach Satz 23, dadurch gekennzeichnet, dass der Kopplungsabschnitt (26) spiegelsymmetrisch bezogen auf eine die Längsachse (12) enthaltende zweite Spiegelebene (114) ausgebildet ist.
25. Medizinische Drehmomentbegrenzungsvorrichtung nach Satz 23 oder 24, dadurch gekennzeichnet, dass der Kopplungsabschnitt (26) N Kopplungselemente (102) umfasst und dass eine Symmetrie des Kopplungsabschnitts (26) bezogen auf die Längsachse (12) entsprechend einer Anzahl N der Kopplungselemente (102) N-zählig ist.
26. Medizinische Drehmomentbegrenzungsvorrichtung nach einem der Sätze 18 bis 25, dadurch gekennzeichnet, dass am mindestens einen Mitnehmerelement (20) in Umfangsrichtung abwechselnd ein Kopplungselement (100) und ein Verformungsabschnitt (88) angeordnet oder ausgebildet sind.
27. Medizinische Drehmomentbegrenzungsvorrichtung nach einem der voranstehenden Sätze, dadurch gekennzeichnet, dass das mindestens eine Mitnehmerelement (20) in Form eines in sich geschlossenen, die Längsachse (12) umgebenden Ringelements (86) ausgebildet ist.
28. Medizinische Drehmomentbegrenzungsvorrichtung nach einem der voranstehenden Sätze, dadurch gekennzeichnet, dass die erste Komponente (14) und die zweite Komponente (16) in einer Sicherungsstellung unverlierbar aneinander gesichert sind.
29. Medizinische Drehmomentbegrenzungsvorrichtung nach Satz 28, dadurch gekennzeichnet, dass die medizinische Drehmomentbegrenzungsvorrichtung (10) eine Sicherungseinrichtung (116) zum Sichern der ersten Komponente (14) und der zweiten Komponente (16) in der Sicherungsstellung gegen eine Bewegung relativ zueinander in einer Richtung parallel zur Längsachse (12) umfasst.
30. Medizinische Drehmomentbegrenzungsvorrichtung nach Satz 29, dadurch gekennzeichnet, dass die Sicherungseinrichtung (116) ausgebildet ist zum Begrenzen einer Bewegung des mindestens einen Mitnehmerelements (20) und der ersten Komponente (14) in einer Richtung parallel zur Längsachse (12).
31. Medizinische Drehmomentbegrenzungsvorrichtung nach Satz 30, dadurch gekennzeichnet, dass die Sicherungseinrichtung zwei aufeinander zu weisende Anschläge (60, 64) für das mindestens eine Mitnehmerelement (20) umfasst, wobei insbesondere ein erster Anschlag (60) an der ersten Komponente (14) angeordnet oder ausgebildet ist und ein zweiter Anschlag (64) an der zweiten Komponente (16) angeordnet oder ausgebildet ist.
32. Medizinische Drehmomentbegrenzungsvorrichtung nach Satz 31, dadurch gekennzeichnet, dass einer der Anschläge (60) in Form eines in radialer Richtung vorspringenden Ringflansches (30) ausgebildet ist.
33. Medizinische Drehmomentbegrenzungsvorrichtung nach Satz 31 oder 32, dadurch gekennzeichnet, dass einer der Anschläge (60, 64) durch das geschlossene Ende (106) der Kopplungsnut (38) gebildet ist.
34. Medizinische Drehmomentbegrenzungsvorrichtung nach einem der Sätze 29 bis 33, dadurch gekennzeichnet, dass die Sicherungseinrichtung (116) mindestens ein Sicherungselement (118) umfasst zum Begrenzen einer Bewegung der ersten Komponente (14) und der zweiten Komponente (16) in einer Richtung parallel zur Längsachse (12).
35. Medizinische Drehmomentbegrenzungsvorrichtung nach Satz 34, dadurch gekennzeichnet, dass das mindestens eine Sicherungselement (118) einen Sicherungsring (120) umfasst und dass der Sicherungsring (120) in der Sicherungsstellung in die Längsachse umgebende Ringaufnahmen (44, 56) an der ersten und der zweiten Komponente (14, 16) eingreift.
36. Medizinische Drehmomentbegrenzungsvorrichtung nach Satz 35, dadurch gekennzeichnet, dass der Sicherungsring (120) offen ausgebildet ist und zwei aufeinander zu weisende Sicherungsringenden (122) aufweist.
37. Medizinische Drehmomentbegrenzungsvorrichtung nach Satz 35 oder 36, dadurch gekennzeichnet, dass die Ringaufnahme (44) an der ersten Komponente (14) und die Kopplungsnut (38) sich kreuzen.
38. Medizinische Drehmomentbegrenzungsvorrichtung nach einem der voranstehenden Sätze, dadurch gekennzeichnet, dass an der ersten Komponente (14) ein Werkzeugkopplungsglied (32) angeordnet oder ausgebildet ist zum kraft- und/oder formschlüssigen in Eingriff Bringen mit einem ein Drehmoment in die erste Komponente (14) einleitenden medizinischen Werkzeug.
39. Medizinische Drehmomentbegrenzungsvorrichtung nach Satz 38, dadurch gekennzeichnet, dass das Werkzeugkopplungsglied (32) in Form eines Werkzeugkopplungsvorsprungs (34) oder in Form einer Werkzeugkopplungsaufnahme ausgebildet ist.
40. Medizinische Drehmomentbegrenzungsvorrichtung nach Satz 39, dadurch gekennzeichnet, dass die Werkzeugkopplungsaufnahme schlitz-, kreuz- oder sternförmig ausgebildet ist.
41. Medizinische Drehmomentbegrenzungsvorrichtung nach einem der Sätze 38 bis 40, dadurch gekennzeichnet, dass eine Kontur des Werkzeugkopplungsglieds (32) in Form eines Vielkant (36) oder in Form eines Vielrund ausgebildet ist.
42. Medizinische Drehmomentbegrenzungsvorrichtung nach einem der voranstehenden Sätze, dadurch gekennzeichnet, dass an der zweiten Komponente (16) ein Gewindeabschnitt (128) angeordnet oder ausgebildet ist, insbesondere in Form eines Innengewindes (130) oder eines Außengewindes (134).
43. Medizinische Drehmomentbegrenzungsvorrichtung nach einem der voranstehenden Sätze, dadurch gekennzeichnet, dass die erste Komponente (14) und/oder die zweite Komponente (16) hülsenförmig ausgebildet sind.
44. Medizinische Drehmomentbegrenzungsvorrichtung nach einem der voranstehenden Sätze, dadurch gekennzeichnet, dass die Drehmomentbegrenzungsvorrichtung (10) aus einem oder mehreren sterilisierbaren Werkstoffen ausgebildet ist.
45. Medizinische Drehmomentbegrenzungsvorrichtung nach einem der voranstehenden Sätze, dadurch gekennzeichnet, dass die medizinische Drehmomentbegrenzungsvorrichtung (10) in Form einer medizinischen Mutter (126), einer medizinischen Schraube (132) oder eines medizinischen Drehmomentbegrenzungsschlüssels (140) ausgebildet ist.

Die nachfolgende Beschreibung einer bevorzugten Ausführungsform der Erfindung dient im Zusammenhang mit der Zeichnung der näheren Erläuterung. Es zeigen:
- Figur 1:: eine Explosionsdarstellung eines ersten Ausführungsbeispiels einer medizinischen Drehmomentbegrenzungsvorrichtung;
- Figur 2:: eine Längsschnittansicht der Anordnung aus Figur 1 in einer zusammengesetzten Stellung;
- Figur 3:: eine Schnittansicht längs Linie 3-3 in Figur 2;
- Figur 4:: eine Seitenansicht einer ersten Komponente der Anordnung aus Figur 1 mit mehreren Mitnehmerelementen;
- Figur 5:: eine Schnittansicht längs Linie 5-5 in Figur 4;
- Figur 6:: eine Draufsicht auf die Anordnung in Figur 4 in Richtung des Pfeils A;
- Figur 7:: eine perspektivische Explosionsdarstellung der Anordnung aus Figur 4;
- Figur 8:: eine Schnittansicht ähnlich Figur 2 eines weiteren Ausführungsbeispiels einer medizinischen Drehmomentbegrenzungsvorrichtung in Form einer medizinischen Mutter;
- Figur 9:: eine Schnittansicht ähnlich Figur 2 eines weiteren Ausführungsbeispiels einer medizinischen Drehmomentbegrenzungsvorrichtung in Form einer medizinischen Schraube; und
- Figur 10:: eine Schnittansicht ähnlich Figur 2 eines weiteren Ausführungsbeispiels einer medizinischen Drehmomentbegrenzungsvorrichtung in Form eines medizinischen Drehmomentbegrenzungsschlüssels.

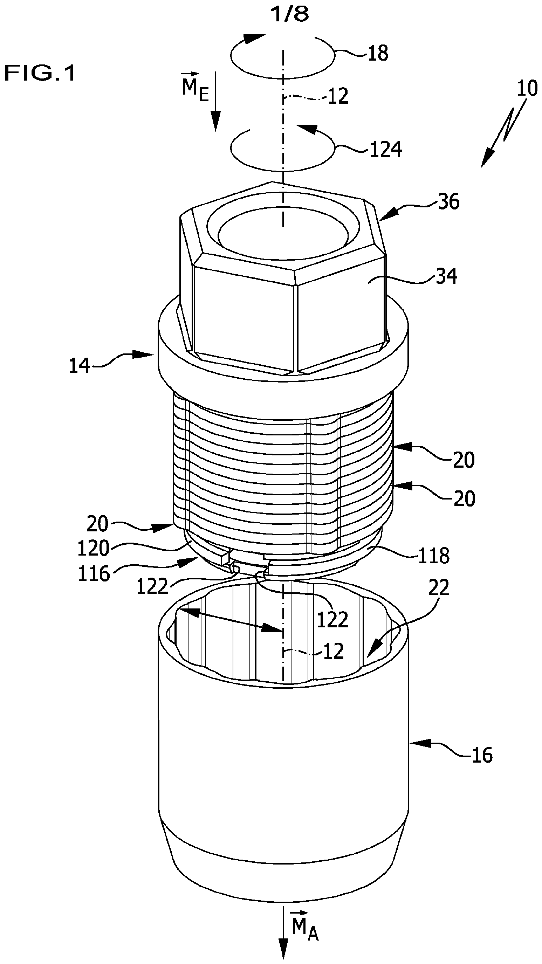

Ein erstes Ausführungsbeispiel einer medizinischen Drehmomentbegrenzungsvorrichtung ist schematisch in Figur 1 dargestellt und insgesamt mit dem Bezugszeichen 10 bezeichnet.

Die Drehmomentbegrenzungsvorrichtung definiert eine Längsachse 12 und umfasst eine erste Komponente 14 und eine zweite Komponente 16. Die beiden Komponenten 14 und 16 sind jeweils hülsenförmig ausgebildet.

Wie nachfolgend im Einzelnen noch näher beschrieben wird, sind die erste Komponente 14 und die zweite Komponente 16 zusammenwirkend ausgebildet zum Übertragen eines auf die erste Komponente 14 wirkenden Drehmoments M_{E} auf die zweite Komponente 16 derart, dass bei einem in einer Anzugsdrehrichtung 18 auf die erste Komponente 14 wirkenden Drehmoment M_{E}, welches kleiner als ein Grenzdrehmoment Meist, die erste Komponente 14 und die zweite Komponente 16 in der Anzugsdrehrichtung 18 relativ zueinander um die Längsachse 12 unverdrehbar sind. Bei einem in der Anzugsdrehrichtung 18 auf die erste Komponente 14 wirkenden Drehmoment M_{E}, welches mindestens dem Drehmoment M_{G} entspricht, sind die erste Komponente 14 und die zweite Komponente 16 in der Anzugsdrehrichtung 18 relativ zueinander um die Längsachse 18 verdrehbar.

Um die beschriebene Funktionsweise der Drehmomentbegrenzungsvorrichtung 10 zu ermöglichen, sind an der ersten Komponente 14 mehrere verformbare Mitnehmerelemente 20 angeordnet. Die Mitnehmerelemente 20 wirken mit einer an der zweiten Komponente 16 ausgebildeten Verformungsfläche 22 zusammen. Die Verformungsfläche 22 ist derart ausgebildet, dass ein Abstand 24 der Verformungsfläche 24 von der Längsachse 12 sich in Umfangsrichtung ändert, oder mit anderen Worten, dass der Abstand 24 der Verformungsfläche 22 von der Längsachse 12 eine Funktion eines Umfangswinkels bezogen auf die Längsachse 12 ist.

Die erste Komponente 14 umfasst einen hülsenförmigen Kopplungsabschnitt 26, welcher sich ausgehend von einem distalen Ende 28 bis zu einem in radialer Richtung von der Längsachse 12 weg weisend vorstehenden Ringflansch 30 erstreckt. Proximalseitig des Ringflansches 30 ist an der ersten Komponente 14 ein Werkzeugkopplungsglied 32 angeordnet beziehungsweise ausgebildet zum kraft- und/oder formschlüssigen in Eingriff bringen mit einem ein Drehmoment M_{E} in die erste Komponente 14 einleitenden, in den Figuren nicht dargestellten medizinischen Werkzeugs.

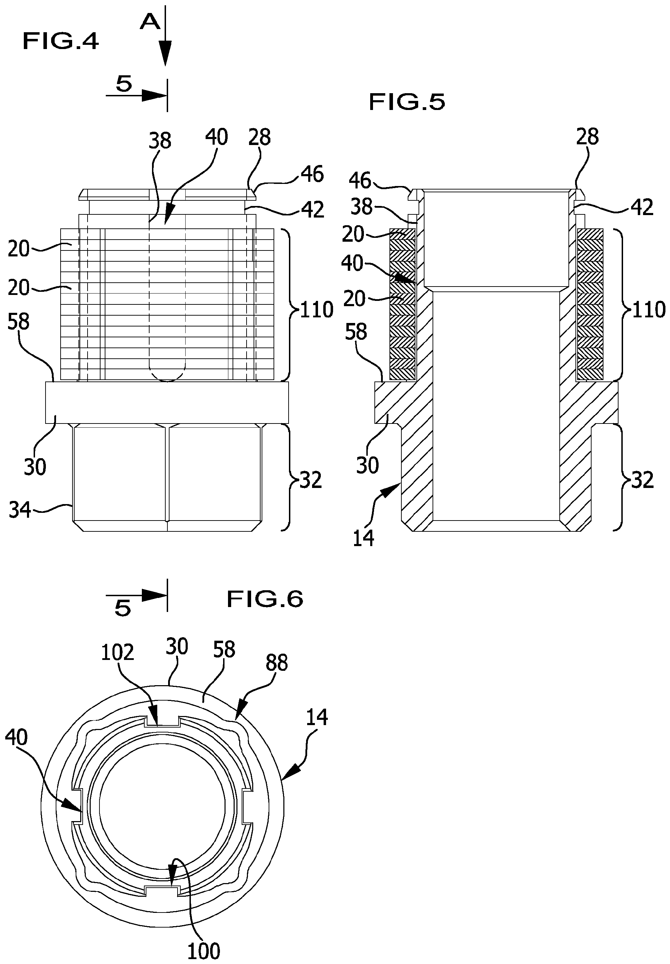

Das Werkzeugkopplungsglied 32 ist bei dem in den Figuren 1 bis 7 dargestellten Ausführungsbeispiel in Form eines Werkzeugkopplungsvorsprungs 34 ausgebildet. Eine Kontur des Werkzeugkopplungsglieds 32 definiert einen Vielkant 36, bei dem in den Figuren dargestellten Ausführungsbeispiel einen Sechskant.

Bei weiteren Ausführungsbeispielen ist das Werkzeugkopplungsglied 32 in Form einer Werkzeugkopplungsaufnahme ausgebildet, welche schlitz-, kreuzoder sternförmig ausgebildet ist.

Bei einem weiteren Ausführungsbeispiel ist die Kontur des Werkzeugkopplungsglieds in Form eines Vielrund ausgebildet.

Ausgehend vom distalen Ende 28 sind außen am Kopplungsabschnitt 26 vier sich parallel zur Längsachse 12 streckende Kopplungsnuten 38 ausgebildet. Sie bilden jeweils eine Kopplungsaufnahme 40. Die vier Kopplungsaufnahmen 40 sind gleichmäßig über einen Umfang des Kopplungsabschnitts 26 verteilt.

Ferner ist nahe des distalen Endes 28 außen am Kopplungsabschnitt 26 eine in radialer Richtung von der Längsachse 12 weg weisende die Ringnut 42 ausgebildet, welche eine erste Ringaufnahme 44 bildet. Mithin umgibt die erste Ringaufnahme 44 die Längsachse 12.

Ausgehend vom distalen Ende 28 ist eine von der Längsachse 12 weg weisende Außenfläche des Kopplungsabschnitts 26 etwas abgeschrägt, und zwar derart, dass sich ein Außendurchmesser des Kopplungsabschnitts 26 in Richtung auf das distale Ende 28 verjüngt. Mithin wird auf diese Weise eine ringförmige Aufgleitfläche 26 ausgebildet.

Die zweite Komponente 16 weist ein distales Ende 48 und ein proximales Ende 50 auf. Die Verformungsfläche 22 erstreckt sich ausgehend vom proximalen Ende 50 bis zu einer in proximaler Richtung weisenden ringförmigen Anschlagfläche 52, welche sich von der Verformungsfläche 22 etwas in Richtung auf die Längsachse 12 hin erstreckt.

Hiervon etwas beabstandet ist in distaler Richtung eine in Richtung auf die Längsachse 12 hin geöffnete Ringnut 54 ausgebildet, welche eine zweite Ringaufnahme 56 definiert. Die zweite Komponente 16 ist so bemessen, dass sie mit ihrem proximalen Ende 50 über das distale Ende 28 der ersten Komponente 14 geschoben werden kann, bis das proximale Ende 50 an einer in distaler Richtung weisenden, ringförmigen Anschlagfläche 58 anliegt. Die Anschlagfläche 58 ist am Ringflansch 30 ausgebildet. Der Ringflansch 30 bildet somit einen ersten Anschlag 60. Ein die Anschlagfläche 52 definierender Rücksprung 62 bildet einen zweiten Anschlag 64.

Die Ringaufnahmen 44 und 56 liegen einander gegenüber, wenn das proximale Ende 50 an der Anschlagfläche 58 anliegt.

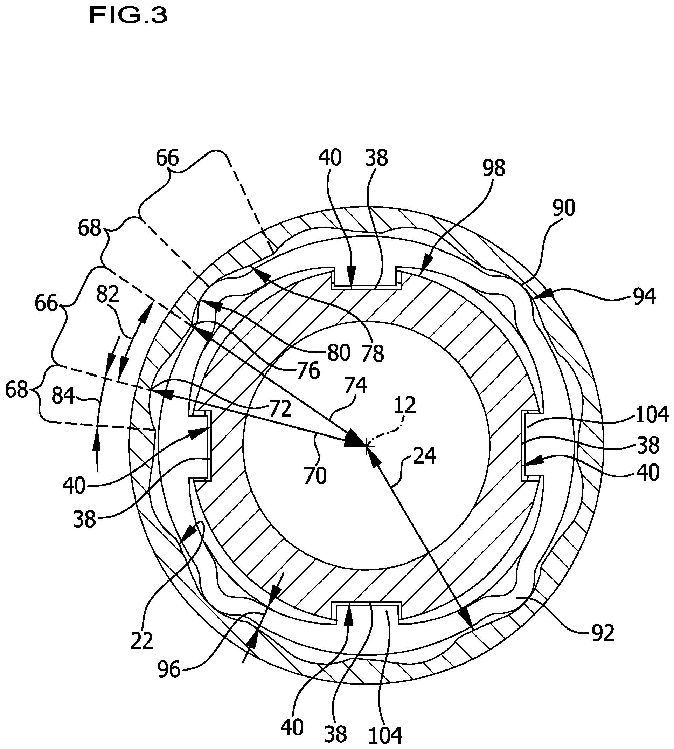

Die Verformungsfläche 22 weist mehrere erste Flächenbereiche 66 und mehrere zweite Flächenbereiche 68 auf. Diese erstrecken sich jeweils parallel zur Längsachse 12. Wie insbesondere in Figur 3 gut zu erkennen, ändert sich der Abstand 24 in Umfangsrichtung in Abhängigkeit von einem Umfangswinkel. Der Abstand 24 ändert sich derart, dass er einen maximalen Abstand 70 längs einer sich parallel zur Längsachse 12 erstreckenden Linie 72 und einen minimalen Abstand 74 längs einer sich parallel zur Längsachse 12 erstreckenden Linie 76 definiert. Die ersten Flächenbereiche 66 erstrecken sich zwischen der Linie 72 und der Linie 76 derart, dass der Abstand 24 ausgehend von der Linie 72 zur Linie 76 hin abnimmt. Der erste Flächenbereich 66 bildet so eine in Umfangsrichtung wirkende erste Anlauframpe 78 bezogen auf die Längsachse 12.

Ausgehend von einer Linie 72 erstreckt sich der zweite Flächenbereich 68 in entgegengesetzter Richtung zur nächsten Linie 76 hin. Er definiert eine zweite Anlauframpe 80. Die zweite Anlauframpe 80 erstreckt sich über einen kleineren Umfangswinkel als die erste Anlauframpe 78. Ein erster Umfangswinkel 82, welcher durch die erste Anlauframpe 78 definiert ist, ist etwa doppelt so groß wie ein zweiter Umfangswinkel 84, welche durch die zweite Anlauframpe 80 definiert ist. Mithin ist die erste Anlauframpe 78 flacher als die zweite Anlauframpe 80.

Bei dem in den Figuren 1 bis 7 schematisch dargestellten Ausführungsbeispiel umfasst die Verformungsfläche 22 zwölf erste Flächenbereiche 66 und zwölf zweite Flächenbereiche 68, die sich in der beschriebenen Weise abwechselnd in Umfangsrichtung um die Längsachse 12 erstrecken.

Die Mitnehmerelemente 20 der Drehmomentbegrenzungsvorrichtung 10 sind identisch ausgebildet. Sie sind in Form von in sich geschlossenen, die Längsachse 12 umgebenden Ringelementen 86 ausgebildet, und zwar aus einem Flachmaterial. Die Mitnehmerelemente 20 sind bei dem beschriebenen Ausführungsbeispiel aus einem Flachmaterial durch Stanzen oder Laserschneiden ausgeschnitten.

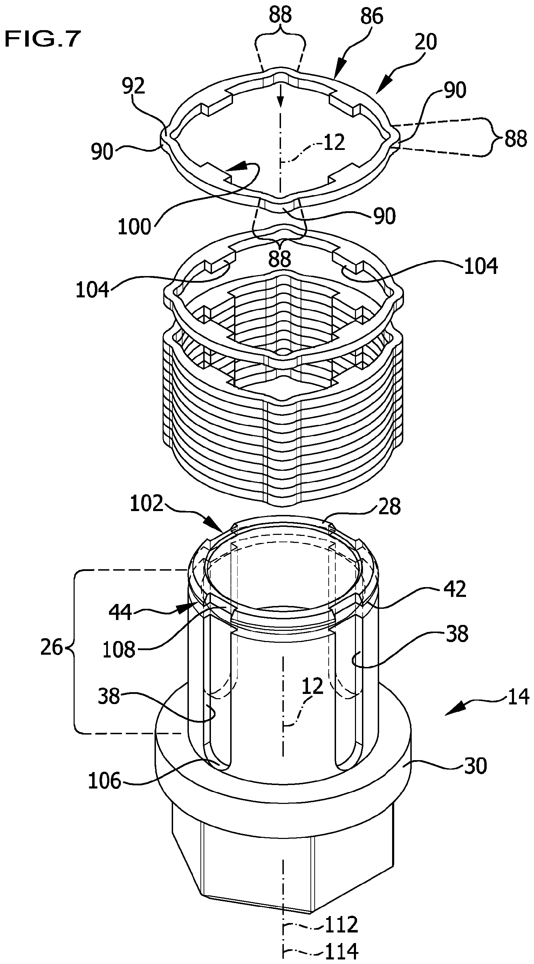

Jedes Mitnehmerelement 20 umfasst mehrere, bei dem in den Figuren dargestellten Ausführungsbeispiel sind es vier, Verformungsabschnitte 88. An jedem Verformungsabschnitt 88 ist eine mit der Verformungsfläche 22 zusammenwirkende Anlagefläche 90 ausgebildet. Jeder Verformungsabschnitt 88 ist ausgehend von einer Grundstellung, wie sie schematisch in Figur 7 dargestellt ist und in welcher kein Drehmoment auf die erste Komponente wirkt, in eine Auslösestellung verformbar, in welcher der Verformungsabschnitt 88 in Richtung auf die Längsachse 12 hin verformt ist. Der Verformungsabschnitt 88 ist in einer Auslösestellung maximal verformt. Diese ist definiert dadurch, dass die Anlagefläche 90 an der Linie 76 anliegt. In der Grundstellung des Verformungsabschnitts 88 weist die Anlagefläche 80 in Richtung auf die Linie 72 hin.

Die Verformungsabschnitte 88 sind in Form federelastischer Elemente 92 ausgebildet. Wie beschrieben sind die Verformungsabschnitte 88 in radialer Richtung bezogen auf die Längsachse 12 federnd ausgebildet. Sie können durch Ausüben einer Kraft in Richtung auf die Längsachse 12 hin ausgelenkt werden und verformen sich elastisch in die Grundstellung, also in eine von der Längsachse 12 weiter entfernte Stellung, zurück, wenn keine Kraft auf die Verformungsabschnitte 88 beziehungsweise die Anlageflächen 90 in radialer Richtung auf die Längsachse 12 hin ausgeübt wird.

Die Verformungsabschnitte 88 umfassen jeweils einen Verformungsvorsprung 94, welcher die Anlagefläche 90 umfasst und bezogen auf die Längsachse 12 in Richtung auf die Verformungsfläche 22 vorstehend beziehungsweise vorgewölbt ausgebildet ist.

Die vier Verformungsabschnitte 88 sind gleichmäßig über einen Umfang des Mitnehmerelements 20 verteilt ausgebildet.

Wie insbesondere in Figur 3 gut zu erkennen, ist der Verformungsabschnitt 88 durch eine Materialschwächung am Mitnehmerelement 20 ausgebildet. Eine Dicke 96 des Ringelements 86 in radialer Richtung ist im Bereich des Verformungsabschnitts 88 am geringsten.

Die Mitnehmerelemente 20 sind mit der ersten Komponente 14 lösbar verbindbar. Um dies zu ermöglichen, umfasst die medizinische Drehmomentbegrenzungsvorrichtung 10 eine Kopplungseinrichtung 98 zum drehfesten Koppeln der Mitnehmerelemente 20 und der ersten Komponente 14 in einer Kopplungsstellung.

Die Kopplungseinrichtung 98 umfasst erste Kopplungselemente 100 und zweite Kopplungselemente 102. Eines der Kopplungselemente 100, 102 ist am Mitnehmerelement 20 angeordnet beziehungsweise ausgebildet, ein anderes der Kopplungselemente 100, 102 ist an der ersten Komponente 14 angeordnet beziehungsweise ausgebildet.

Bei dem in den Figuren 1 bis 7 dargestellten Ausführungsbeispiel sind am Mitnehmerelement 20 vier erste Kopplungselemente 100 ausgebildet. Die erste Komponente 14 umfasst vier zweite Kopplungselemente 102. Die ersten und zweiten Kopplungselemente 100 und 102 stehen in der Kopplungsstellung kraft- und/oder formschlüssig in Eingriff. Dies ist schematisch gut in Figur 3 zu erkennen.

Die ersten Kopplungselemente 100 sind in Form von quaderförmigen, vom Ringelement 86 in Richtung auf die Längsachse 12 vorstehenden Kopplungsvorsprünge 104 ausgebildet. Die zweiten Kopplungselemente 102 sind durch die Kopplungsaufnahmen 40 an der ersten Komponente 14 gebildet. Diese weisen eine Form und Größe auf, dass sie in der Kopplungsstellung jeweils einen Kopplungsvorsprung 104 aufnehmen können.

Wie insbesondere in Figur 7 gut zu erkennen, sind an jedem Mitnehmerelement 20 in Umfangsrichtung abwechselnd ein erstes Kopplungselement 100 und ein Verformungsabschnitt 88 angeordnet beziehungsweise ausgebildet. Mithin umfasst jedes Mitnehmerelement 20 vier erste Kopplungselemente 100 und vier Verformungsabschnitte 88.

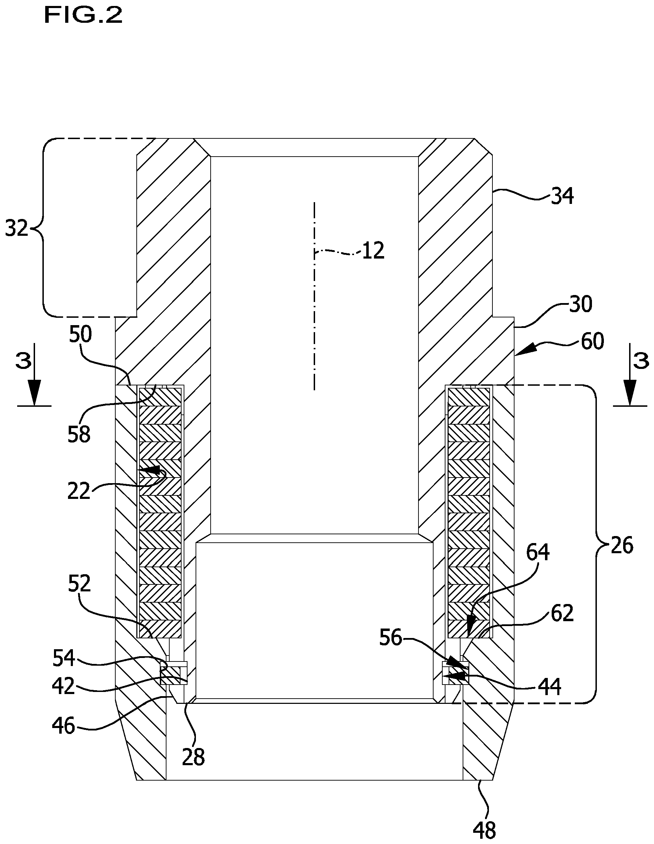

Die Kopplungsaufnahme 40 umfasst ein erstes Ende 106 und ein zweites Ende 108. Das erste Ende 106 ist geschlossen und grenzt an den Ringflansch 30 an. Das zweite Ende 108 ist im Bereich des distalen Endes 28 der ersten Komponente 14 ausgebildet. Das zweite Ende 108 ist offen und ermöglicht es, die Mitnehmerelemente 20 über das distale Ende 28 auf den Kopplungsabschnitt 26 aufzuschieben, und zwar derart, dass die Kopplungsvorsprünge 104 jeweils in eine der vier Kopplungsnuten 38 eingreifen. Die Mitnehmerelemente 20 liegen dann, wie gut in Figur 2 zu erkennen, mit parallel zueinander verlaufenden Seitenflächen, die sich senkrecht zur Längsachse 12 erstrecken, aneinander an und bilden ein Mitnehmerelementepaket 110. In der beschriebenen Weise sind die zweiten Kopplungselemente 102 im Bereich des Kopplungsabschnitts 26 der ersten Komponente 14 angeordnet beziehungsweise ausgebildet.

Die Mitnehmerelemente 20 sind spiegelsymmetrisch bezogen auf eine die Längsachse 12 enthaltende erste Spiegleebene 112 ausgebildet. Der Kopplungsabschnitt 26 ist bezogen auf eine die Längsachse 12 enthaltende zweite Spiegelebene 114 spiegelsymmetrisch ausgebildet. Das in den Figuren 1 bis 7 dargestellte Ausführungsbeispiel der Drehmomentbegrenzungsvorrichtung ist derart ausgebildet, dass es spiegelsymmetrisch zu vier ersten Spiegelebenen 112 und zu vier zweiten Spiegelebenen 114 ist.

Bei dem in den Figuren 1 bis 7 dargestellten Ausführungsbeispiel der Drehmomentbegrenzungsvorrichtung 10 entspricht eine Anzahl M der ersten Flächenbereiche 66 mindestens einer Anzahl N der Verformungsabschnitte 88. Bei dem in den Figuren dargestellten Ausführungsbeispiel entspricht die Anzahl M der ersten Flächenbereiche 66 einem ganzzahligen Vielfachen der Anzahl N der Verformungsabschnitte 88.

Beim dargestellten und beschriebenen Ausführungsbeispiel ist die Anzahl M der ersten Flächenbereiche 66 wie beschrieben M = 12. Die Anzahl N der Verformungsabschnitte ist bei dem Ausführungsbeispiel N = 4.

Der Kopplungsabschnitt 26 umfasst wie beschrieben vier zweite Kopplungselemente 102. Eine Symmetrie des Kopplungsabschnitts 26 bezogen auf die Längsachse 12 ist daher entsprechend der Anzahl der Kopplungselemente 102 vier-zählig. Dies bedeutet, dass der Kopplungsabschnitt 26 durch Rotation um die Längsachse 12 um einen Winkel, welcher einem Viertel von 360° entspricht, in sich selbst überführt werden kann. In analoger Weise ist auch eine Symmetrie der Mitnehmerelemente 20 bezogen auf die Längsachse 12 vier-zählig.

Die erste Komponente 14 und die zweite Komponente 16 sind in einer Sicherungsstellung unverlierbar aneinander gesichert. Hierfür umfasst die medizinische Drehmomentbegrenzungsvorrichtung 10 eine Sicherungseinrichtung 116 zum Sichern der ersten Komponente 14 und der zweiten Komponente 16 in der Sicherungsstellung gegen eine Bewegung relativ zueinander in einer Richtung parallel zur Längsachse 12.

Die Sicherungseinrichtung 116 umfasst ein Sicherungselement 118 zum Begrenzen einer Bewegung der ersten Komponente 14 und der zweiten Komponente 16 in einer Richtung parallel zur Längsachse 12, und zwar sowohl voneinander weg als auch aufeinander zu. Das Sicherungselement 118 ist in Form eines Sicherungsrings 120 ausgebildet und greift in der Sicherungsstellung sowohl in die erste Ringaufnahme 44 als auch in die zweite Ringaufnahme 46 ein, die an der ersten Komponente 14 und der zweiten Komponente 16 ausgebildet sind.

Um eine Montage der Drehmomentbegrenzungsvorrichtung 10 zu ermöglichen, ist der Sicherungsring 120 offen ausgebildet und weist zwei aufeinander zu weisende Sicherungsringenden 122 auf.

Die erste Ringaufnahme 44 und die Kopplungsnuten 38 kreuzen sich. Dies ist insbesondere gut in Figur 7 zu erkennen.

In der Sicherungsstellung, die insbesondere in Figur 2 dargestellt ist, liegt die Anschlagfläche 58 am proximalen Ende 50 an. Die Mitnehmerelemente 20 sind zwischen der Anschlagfläche 58 und der Anschlagfläche 52 gegen eine Bewegung in einer Richtung parallel zur Längsachse 12 gesichert. Mithin ist also die Sicherungseinrichtung 116 ausgebildet zum Begrenzen einer Bewegung der Mitnehmerelemente 20 und der ersten Komponente 14 relativ zueinander in einer Richtung parallel zur Längsachse 12.

Wird in die erste Komponente 14 ein Drehmoment M_{E} in der Anzugsdrehrichtung 18 eingeleitet, wirken die Verformungsabschnitte 88 und die ersten Flächenbereiche 66 miteinander zusammen. Das Drehmoment M_{E} kann bis zum Grenzdrehmoment erhöht werden. Bis dahin verformt sich der Verformungsabschnitt 88 durch Aufgleiten an der ersten Anlauframpe 78 in Richtung auf die Längsachse 12 hin. Ist das Drehmoment in der Anzugsdrehrichtung 18 größer als das Grenzdrehmoment M_{G}, können die Verformungsabschnitte 88 über die Linien 76 hinweg bewegt werden in den zweiten Flächenbereich 68 hinein, sodass die elastisch ausgelenkten Verformungsabschnitte 88 wieder in die Grundstellung zurückfedern können.

In einer Lösedrehrichtung 124, die der Anzugsdrehrichtung 18 entgegengesetzt gerichtet ist, wirken die Verformungsabschnitte 88 mit den zweiten Flächenbereichen 68 zusammen. Da die zweite Anlauframpe 80 viel steiler ist als die erste Anlauframpe 78, ist es nahezu unmöglich, selbst bei Überschreiten des Grenzdrehmoments M_{G}, die Verformungsabschnitte 88 derart zu verformen, dass sie über die Linien 76 hinweg in den ersten Flächenbereich 66 zurück bewegt werden können.

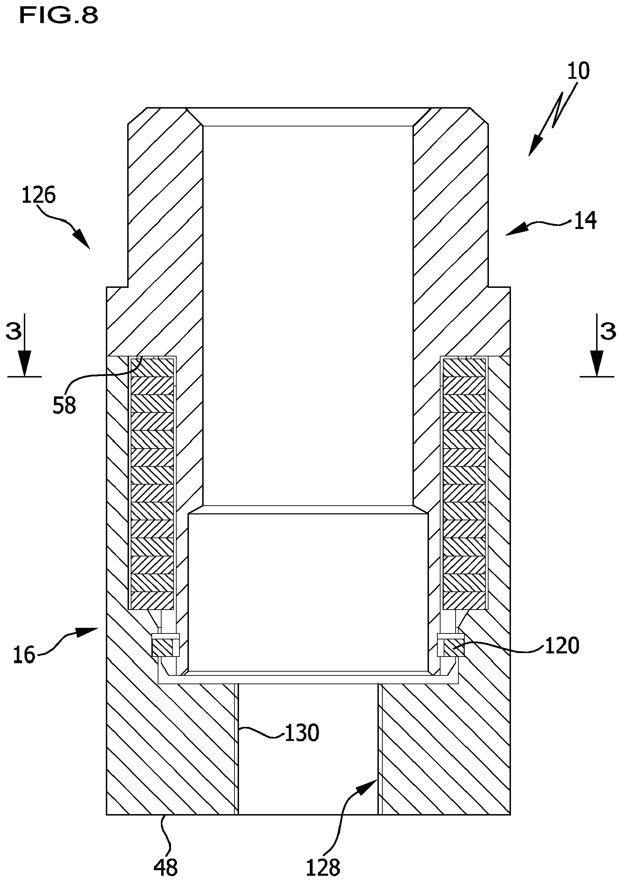

Ein weiteres Ausführungsbeispiel einer medizinischen Drehmomentbegrenzungsvorrichtung 10 ist schematisch in Figur 8 in Form einer medizinischen Mutter 126 dargestellt.

Die Mutter 126 unterscheidet sich von der Drehmomentbegrenzungsvorrichtung der Figuren 1 bis 7 dadurch, dass an der zweiten Komponente 16 ausgehend vom distalen Ende 48 ein Gewindeabschnitt 128 in Form eines Innengewindes 130 ausgebildet ist. Die Mutter 126 bildet somit eine Drehmomentbegrenzungsmutter. Ein in die erste Komponente 14 eingeleitetes Drehmoment M_{E} kann nur wie oben beschrieben bis zum Grenzdrehmoment M_{G} auf die zweite Komponente 16 und damit auf den Gewindeabschnitt 128 übertragen werden.

Im Übrigen ist die Mutter 126 identisch ausgebildet wie die oben beschriebene Drehmomentbegrenzungsvorrichtung 10 in Verbindung mit den Figuren 1 bis 7.

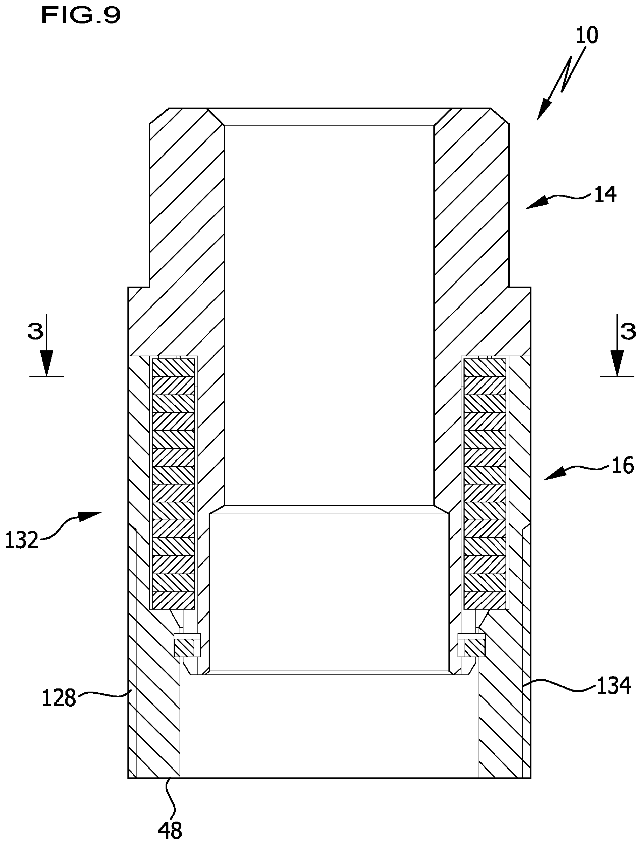

Figur 9 zeigt schematisch ein weiteres Ausführungsbeispiel einer Drehmomentbegrenzungsvorrichtung 10, und zwar in Form einer medizinischen Schraube 132. Hier ist an der zweiten Komponente 16 ausgehend vom distalen Ende 48 ein Gewindeabschnitt 128 in Form eines Außengewindes 134 ausgebildet. Wird auf die erste Komponente 14 der Schraube 132 ein Drehmoment M_{E} eingeleitet, wird es wie oben beschrieben nur bis zum Grenzdrehmoment M_{G} auf die zweite Komponente 16 übertragen. Damit kann der Gewindeabschnitt 128 nur mit einem maximalen Drehmoment, welches dem Grenzdrehmoment M_{G} entspricht, in ein Innengewinde eines weiteren Bauteils eingeschraubt werden.

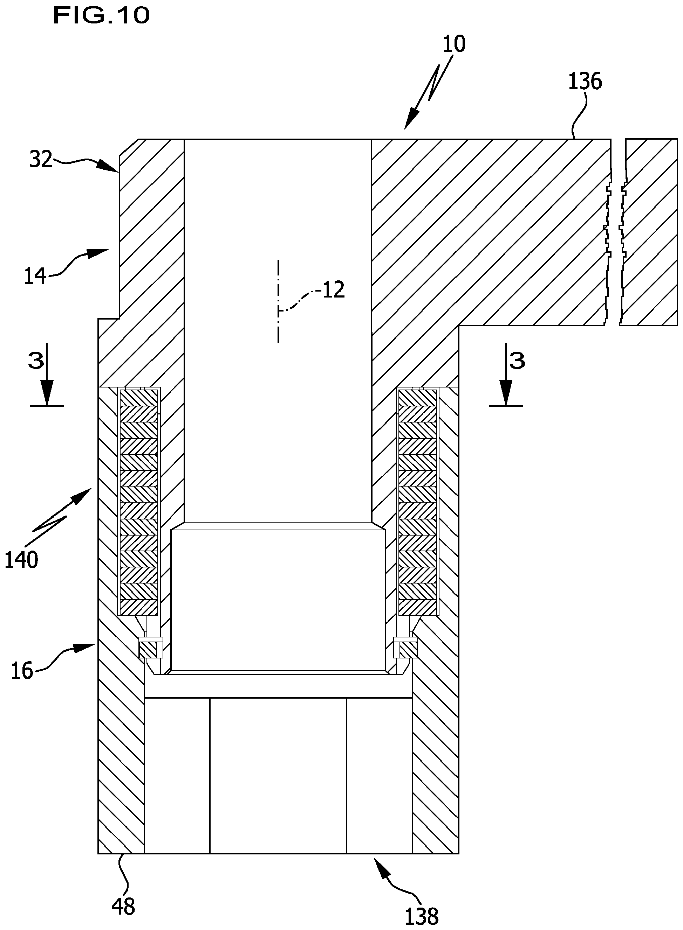

Ein weiteres Ausführungsbeispiel einer medizinischen Drehmomentbegrenzungsvorrichtung 10 ist schematisch in Figur 10 dargestellt.

Die Drehmomentbegrenzungsvorrichtung 10 gemäß Figur 10 unterscheidet sich vom Ausführungsbeispiel der Figuren 1 bis 7 dadurch, dass an der ersten Komponente 14 im Bereich des Werkzeugkopplungsglieds 32 ein quer, nämlich senkrecht bezogen auf die Längsachse 12 abstehender Hebelarm 136 angeordnet beziehungsweise ausgebildet ist. Ferner ist an der zweiten Komponente 16 ausgehend vom distalen Ende 48 ein Kopplungsglied 138 angeordnet beziehungsweise ausgebildet, um mit einem Werkzeugkopplungsglied in Eingriff gebracht zu werden, beispielsweise einem Schraubenkopf einer Schraube. In Figur 10 ist das Kopplungsglied 138 in Form eines Innenmehrkant schematisch dargestellt.

Das Kopplungsglied 138 weist bei einem weiteren Ausführungsbeispiel eine Außenkontur auf, die zu einem Werkzeugkopplungsglied mit einer Innenkontur, beispielsweise einem Innenvielkant oder einem Innenvielrund, korrespondiert und daher mit diesem formschlüssig in Eingriff gebracht werden kann.

Der Drehmomentbegrenzungsschlüssel 140 ermöglicht es, ein über die erste Komponente 14 eingeleitetes Drehmoment M_{E} hochpräzise nur bis zu einem Grenzdrehmoment M_{G} auf die zweite Komponente 16 und damit auf das Kopplungsglied 138 zu übertragen.

Alle beschriebenen Ausführungsbeispiele von Drehmomentbegrenzungsvorrichtungen 10 sind für den medizinischen und damit insbesondere sterilen Einsatz aus einem oder mehreren sterilisierbaren Werkstoffen ausgebildet, beispielsweise einem Instrumentenstahl.

Wie eingangs beschrieben ermöglichen es die oben in Zusammenhang mit den Figuren 1 bis 10 beschriebenen Drehmomentbegrenzungsvorrichtungen 10, das jeweilige Grenzdrehmoment M_{G} hochpräzise festzulegen. Die Mitnehmerelemente 20 können hierfür unter Berücksichtigung der jeweiligen Fertigungstoleranzen so gewählt werden, dass sich eine Gesamttoleranz, die sich aus der Summe der Fertigungstoleranzen aller Mitnehmerelemente 20 der Drehmomentbegrenzungsvorrichtung 10 ergibt, gegenseitig aufheben oder im Wesentlichen aufheben.

### Bezugszeichenliste

- 10: Drehmomentbegrenzungsvorrichtung
- 12: Längsachse
- 14: erste Komponente
- 16: zweite Komponente
- 18: Anzugsdrehrichtung
- 20: Mitnehmerelement
- 22: Verformungsfläche
- 24: Abstand
- 26: Kopplungsabschnitt
- 28: distales Ende
- 30: Ringflansch
- 32: Werkzeugkopplungsglied
- 34: Werkzeugkopplungsvorsprung
- 36: Vielkant
- 38: Kopplungsnut
- 40: Kopplungsaufnahme
- 42: Ringnut
- 44: erste Ringaufnahme
- 46: Aufgleitfläche
- 48: distales Ende
- 50: proximales Ende
- 52: Anschlagfläche
- 54: Ringnut
- 56: zweite Ringaufnahme
- 58: Anschlagfläche
- 60: erster Anschlag
- 62: Rücksprung
- 64: zweiter Anschlag
- 66: erster Flächenbereich
- 68: zweiter Flächenbereich
- 70: maximaler Abstand
- 72: Linie
- 74: minimaler Abstand
- 76: Linie
- 78: erste Anlauframpe
- 80: zweite Anlauframpe
- 82: erster Umfangswinkel
- 84: zweiter Umfangswinkel
- 86: Ringelement
- 88: Verformungsabschnitt
- 90: Anlagefläche
- 92: Element
- 94: Verformungsvorsprung
- 96: Dicke
- 98: Kopplungseinrichtung
- 100: erstes Kopplungselement
- 102: zweites Kopplungselement
- 104: Kopplungsvorsprung
- 106: erstes Ende
- 108: zweites Ende
- 110: Mitnehmerelementpaket
- 112: erste Spiegelebene
- 114: zweite Spiegelebene
- 116: Sicherungseinrichtung
- 118: Sicherungselement
- 120: Sicherungsring
- 122: Sicherungsringende
- 124: Lösedrehrichtung
- 126: Mutter
- 128: Gewindeabschnitt
- 130: Innengewinde
- 132: Schraube
- 134: Außengewinde
- 136: Hebelarm
- 138: Kopplungsglied
- 140: Drehmomentbegrenzungsschlüssel

## Patentansprüche

1. Medizinische Drehmomentbegrenzungsvorrichtung (10), welche eine Längsachse (12) definiert und eine erste Komponente (14) und eine zweite Komponente (16) umfasst, wobei die erste Komponente (14) und die zweite Komponente (16) zusammenwirkend ausgebildet sind zum Übertragen eines auf die erste Komponente (14) wirkenden Drehmoments auf die zweite Komponente (16) derart, dass bei einem in einer Anzugsdrehrichtung (18) auf die erste Komponente (14) wirkenden Drehmoment, welches kleiner als ein Grenzdrehmoment ist, die erste Komponente (14) und die zweite Komponente (16) in der Anzugsdrehrichtung (18) relativ zueinander um die Längsachse (12) unverdrehbar und bei einem in der Anzugsdrehrichtung (18) auf die erste Komponente (14) wirkenden Drehmoment, welches mindestens dem Grenzdrehmoment entspricht, die erste Komponente (14) und die zweite Komponente (16) in der Anzugsdrehrichtung relativ zueinander um die Längsachse (12) verdrehbar sind, **dadurch gekennzeichnet, dass** an der ersten Komponente (14) mindestens ein verformbares Mitnehmerelement (20) angeordnet oder ausgebildet ist, dass an der zweiten Komponente (16) eine mit dem Mitnehmerelement (20) zusammenwirkende Verformungsfläche (22) angeordnet oder ausgebildet ist und dass sich ein Abstand (24) der Verformungsfläche (22) von der Längsachse (12) in Umfangsrichtung ändert.

2. Medizinische Drehmomentbegrenzungsvorrichtung nach Anspruch 1, **dadurch gekennzeichnet, dass** das mindestens eine Mitnehmerelement (20) mindestens einen Verformungsabschnitt (88) umfasst, dass am Verformungsabschnitt (88) eine mit der Verformungsfläche (22) zusammenwirkende Anlagefläche (90) angeordnet oder ausgebildet ist und dass der Verformungsabschnitt (88) ausgehend von einer Grundstellung, in welcher kein Drehmoment auf die erste Komponente (14) wirkt, in eine Auslösestellung verformbar ist, in welcher auf die erste Komponente (14) das Grenzdrehmoment wirkt,
wobei insbesondere der mindestens eine Verformungsabschnitt (88)
a) in der Auslösestellung maximal verformt ist
und/oder
b) ein federelastisches Element (92) umfasst oder in Form eines federelastischen Elements (92) ausgebildet ist
und/oder
c) in radialer Richtung bezogen auf die Längsachse (12) federnd ausgebildet ist
und/oder
d) einen Verformungsvorsprung (94) umfasst, dass der Verformungsvorsprung (94) die Anlagefläche (90) umfasst und bezogen auf die Längsachse (12) in Richtung auf die Verformungsfläche (22) vorstehend oder vorgewölbt ausgebildet ist.

3. Medizinische Drehmomentbegrenzungsvorrichtung nach einem der voranstehenden Ansprüche, **dadurch gekennzeichnet, dass** die Verformungsfläche (22) mindestens einen ersten Flächenbereich (66) und mindestens einen zweiten Flächenbereich (68) umfasst, dass der erste Flächenbereich (66) mit dem mindestens einen Verformungsabschnitt (88) in der Anzugsdrehrichtung (18) zusammenwirkend ausgebildet ist und dass der mindestens eine zweite Flächenbereich (68) in einer der Anzugsdrehrichtung (18) entgegengesetzt gerichteten Lösedrehrichtung (124) mit dem mindestens einen Verformungsabschnitt (88) zusammenwirkend ausgebildet ist,
wobei insbesondere
a) der mindestens eine erste Flächenbereich (66) eine in Umfangsrichtung wirkende erste Anlauframpe (78) bezogen auf die Längsachse (12) für den mindestens einen Verformungsabschnitt (88) bildet, dass der mindestens eine zweite Flächenbereich (68) eine in Umfangsrichtung wirkende zweite Anlauframpe (80) bezogen auf die Längsachse (12) für den mindestens einen Verformungsabschnitt (88) bildet und dass die erste Anlauframpe (78) flacher ist als die zweite Anlauframpe (80),
und/oder
b) die Verformungsfläche (22) mehrere erste und zweite Flächenbereiche (66, 68) umfasst und dass die ersten und zweiten Flächenbereiche (66, 68) in Umfangsrichtung abwechselnd angeordnet oder ausgebildet sind,
wobei insbesondere eine Anzahl M der ersten Flächenbereiche (66) mindestens einer Anzahl N der Verformungsabschnitte (88) entspricht, insbesondere einem ganzzahligen Vielfachen.

4. Medizinische Drehmomentbegrenzungsvorrichtung nach einem der voranstehenden Ansprüche, **dadurch gekennzeichnet, dass** der mindestens eine Verformungsabschnitt (88) durch eine Materialschwächung am mindestens einen Mitnehmerelement (20) ausgebildet ist.

5. Medizinische Drehmomentbegrenzungsvorrichtung nach einem der voranstehenden Ansprüche, **dadurch gekennzeichnet, dass** die Drehmomentbegrenzungsvorrichtung (10) mehrere Mitnehmerelemente (20) umfasst, insbesondere 2 bis 20 Mitnehmerelemente (20), weiter insbesondere 6 bis 14 Mitnehmerelemente (20),
wobei insbesondere die mehreren Mitnehmerelemente (20) identisch ausgebildet sind.

6. Medizinische Drehmomentbegrenzungsvorrichtung nach einem der voranstehenden Ansprüche, **dadurch gekennzeichnet, dass** die medizinische Drehmomentbegrenzungsvorrichtung (10) eine Kopplungseinrichtung (98) umfasst zum drehfesten Koppeln des mindestens einen Mitnehmerelements (20) und der ersten Komponente (14) in einer Kopplungsstellung,
wobei insbesondere die Kopplungseinrichtung (98) erste und zweite Kopplungselemente (100, 102) umfasst, dass mindestens ein Kopplungselement (100) der ersten und zweiten Kopplungselemente (100, 102) am mindestens einen Mitnehmerelement (20) angeordnet oder ausgebildet ist, dass mindestens ein Kopplungselement (102) der ersten und zweiten Kopplungselemente (100, 102) an der ersten Komponente (14) angeordnet oder ausgebildet ist und dass die ersten und zweiten Kopplungselemente (110, 102), die am mindestens einen Mitnehmerelement (20) einerseits und an der ersten Komponente (14) andererseits angeordnet oder ausgebildet sind, in der Kopplungsstellung kraft- und/oder formschlüssig in Eingriff stehen.

7. Medizinische Drehmomentbegrenzungsvorrichtung nach Anspruch 6, **dadurch gekennzeichnet, dass** die erste Komponente (14) einen Kopplungsabschnitt (26) umfasst, welcher sich parallel zur Längsachse (12) erstreckt, und dass die von der ersten Komponente (14) umfassten Kopplungselemente (102) im Bereich des Kopplungsabschnitts (26) angeordnet oder ausgebildet sind.

8. Medizinische Drehmomentbegrenzungsvorrichtung nach einem der voranstehenden Ansprüche, **dadurch gekennzeichnet, dass** das mindestens eine Mitnehmerelement (20) in Form eines in sich geschlossenen, die Längsachse (12) umgebenden Ringelements (86) ausgebildet ist.

9. Medizinische Drehmomentbegrenzungsvorrichtung nach einem der voranstehenden Ansprüche, **dadurch gekennzeichnet, dass** die erste Komponente (14) und die zweite Komponente (16) in einer Sicherungsstellung unverlierbar aneinander gesichert sind,
wobei insbesondere die medizinische Drehmomentbegrenzungsvorrichtung (10) eine Sicherungseinrichtung (116) zum Sichern der ersten Komponente (14) und der zweiten Komponente (16) in der Sicherungsstellung gegen eine Bewegung relativ zueinander in einer Richtung parallel zur Längsachse (12) umfasst,
wobei weiter insbesondere die Sicherungseinrichtung (116) mindestens ein Sicherungselement (118) umfasst zum Begrenzen einer Bewegung der ersten Komponente (14) und der zweiten Komponente (16) in einer Richtung parallel zur Längsachse (12).

10. Medizinische Drehmomentbegrenzungsvorrichtung nach einem der voranstehenden Ansprüche, **dadurch gekennzeichnet, dass** an der ersten Komponente (14) ein Werkzeugkopplungsglied (32) angeordnet oder ausgebildet ist zum kraft- und/oder formschlüssigen in Eingriff Bringen mit einem ein Drehmoment in die erste Komponente (14) einleitenden medizinischen Werkzeug.

11. Medizinische Drehmomentbegrenzungsvorrichtung nach Anspruch 10, **dadurch gekennzeichnet, dass**
a) das Werkzeugkopplungsglied (32) in Form eines Werkzeugkopplungsvorsprungs (34) oder in Form einer Werkzeugkopplungsaufnahme ausgebildet ist
und/oder
b) die Werkzeugkopplungsaufnahme schlitz-, kreuz- oder sternförmig ausgebildet ist
und/oder
c) eine Kontur des Werkzeugkopplungsglieds (32) in Form eines Vielkant (36) oder in Form eines Vielrund ausgebildet ist.

12. Medizinische Drehmomentbegrenzungsvorrichtung nach einem der voranstehenden Ansprüche, **dadurch gekennzeichnet, dass** an der zweiten Komponente (16) ein Gewindeabschnitt (128) angeordnet oder ausgebildet ist, insbesondere in Form eines Innengewindes (130) oder eines Außengewindes (134).

13. Medizinische Drehmomentbegrenzungsvorrichtung nach einem der voranstehenden Ansprüche, **dadurch gekennzeichnet, dass** die erste Komponente (14) und/oder die zweite Komponente (16) hülsenförmig ausgebildet sind.

14. Medizinische Drehmomentbegrenzungsvorrichtung nach einem der voranstehenden Ansprüche, **dadurch gekennzeichnet, dass** die Drehmomentbegrenzungsvorrichtung (10) aus einem oder mehreren sterilisierbaren Werkstoffen ausgebildet ist.

15. Medizinische Drehmomentbegrenzungsvorrichtung nach einem der voranstehenden Ansprüche, **dadurch gekennzeichnet, dass** die medizinische Drehmomentbegrenzungsvorrichtung (10) in Form einer medizinischen Mutter (126), einer medizinischen Schraube (132) oder eines medizinischen Drehmomentbegrenzungsschlüssels (140) ausgebildet ist.
